# EUROPEAN PATENT SPECIFICATION

(11) **EP 2 096 985 B1**
(45) Date of publication and mention of the grant of the patent: **04.08.2010**
(21) Application number: 07846423.7
(22) Date of filing: 20.12.2007
(51) Int. Cl.: A61B 5/00, A61B 5/022

(54) **A SYSTEM FOR MEASURING BLOOD PRESSURE IN AN ARTERY**
SYSTEM ZUR MESSUNG DES BLUTDRUCKS IN EINER ARTERIE
SYSTEME DE MESURE DE LA PRESSION SANGUINE DANS UNE ARTERE

(30) Priority: 04.01.2007 DK 200700008
(43) Date of publication of application: 09.09.2009
(73) Proprietor: Sense A/S, 2820 Gentofte (DK)
(72) Inventor: LADING, Lars, DK-4000 Roskilde (DK)
(74) Representative: Hansen, Anders Sandgaard
(86) International application number: PCT/DK2007/000553
(87) International publication number: WO 2008/080399

(56) References cited:
- EP-A- 1 405 592
- EP-A- 1 658 807
- DE-A1-102004 032 579

## Description

### FIELD OF INVENTION

The present invention relates to a system for non-interfering blood pressure measurements. In particular, the invention relates to systems for validating the recording of the blood pressure in relation to the position of the sensor relative to the heart.

### BACKGROUND OF INVENTION

Blood pressure can be measured in a number of ways: by use of an invasive pressure sensor, an oscillometric sensor, an auscultatory sensor and a tonometric sensor. These methods will inevitably affect the state of the patient. It has been reported that a considerable number of measurements performed at the office of a medical doctor or at a hospital are affected by the general circumstances and may be quite erroneous compared to what would have been measured if the patient had not been affected by the medical environment.

US 6,558,335 discloses a sensor that is mounted on the wrist of a person/patient in order to make blood pressure measurements less complicated and less dependent on having the patient in a fixed well defined position.

A system for passive pressure sensing with an implantable device is described in US 6,855,115. Another description of a wireless pressure sensor in system for measuring the pressure in a tube is given in US 7,059,196.

EP-A-1 658 807 describes a blood pressure measuring system with wireless transmission of pressure signals.

However, the measured blood pressure at the wrist of a patient will depend on the position of the wrist in relation to the heart, in particular on the elevation of the sensor relative to the heart and the transceiver.

None of the systems disclosed in the above mentioned documents include validation schemes that relates to the position of the sensor relative to the transceiver.

### SUMMARY OF THE INVENTION

According to the invention a system for measuring blood pressure in an artery by wireless reading of a sensor is provided, the system comprising: a sensor having a sensor antenna, the sensor being mounted in close proximity to an artery and emitting a sensor signal in response to the blood pressure in the artery, and wherein the sensor antenna allows for wireless reading of the sensor signal, and a transceiver having a transceiver antenna and being adapted to wirelessly read the sensor signal from a position close to the heart of the patient. The antenna properties of the sensor antenna and the transceiver antenna are designed so that a reading of the sensor signal can only be performed when the sensor is within a predetermined space relative to the transceiver.

The system according to the invention is a sensor system for measuring the blood pressure with a minimum of interference to the patient. The system consists of a sensing device in the form of a sensor, a transceiver, and a computing and recording device. The sensor is typically placed at the wrist. Preferably the sensor is an extra corporal sensor. The transceiver can wirelessly read the state of the sensor.

Typically, the transceiver is attached to the patient, e.g. by a strap around the waist or the chest and thus, the position of the transceiver in relation to the heart may be well defined. In an embodiment, the transceiver may be incorporated in a bandage and fastened to the skin close to the heart.

In a preferred embodiment of the invention, the sensor antenna is designed to have a direction independent gain and the transceiver antenna is designed to have a directional gain which provides for a large sensitivity when the sensor is within a predetermined space relative to the transceiver and a small sensitivity when the sensor is positioned outside this space.

The wireless coupling between the sensor and the transceiver may be an inductive coupling, a capacitive coupling and/or an electromagnetic coupling. The transceiver may be adapted for handling more than one coupling scheme, e.g. an inductive coupling and an electromagnetic coupling.

In an embodiment of the present invention, the wireless reading of the sensor signal is based on inductive coupling between the sensor and the transceiver. A transceiver coil of the transceiver antenna may be shaped so that the desired antenna characteristic is attained. The transceiver may be adapted such that the transceiver coil may have a loop shape with the largest dimension substantially parallel to the longitudinal axis of the patient. The loop shape may be expressed as an ellipse or a higher order ellipse. The transceiver may comprise one or more coils, such as two, three, four, or more coils. The antenna characteristic of the transceiver antenna may have a cross section substantially forming an ellipse or a higher order ellipse.

Inductive coupling can be exploited by using two coils, e.g. a first coil in the sensor and a second coil in the transceiver. Let us assume that one coil, e.g. the second coil, is powered by an oscillating current. The other coil, e.g. the first coil, is then defined as the passive coil that receives energy from the power coil. In order to obtain a signal from the passive coil the coupling efficiency has to exceed a certain value, which depends on the amplitude and frequency of the current in the power coil, on the modulation effect associated with the passive coil, and on the noise properties of the electronic circuitry.

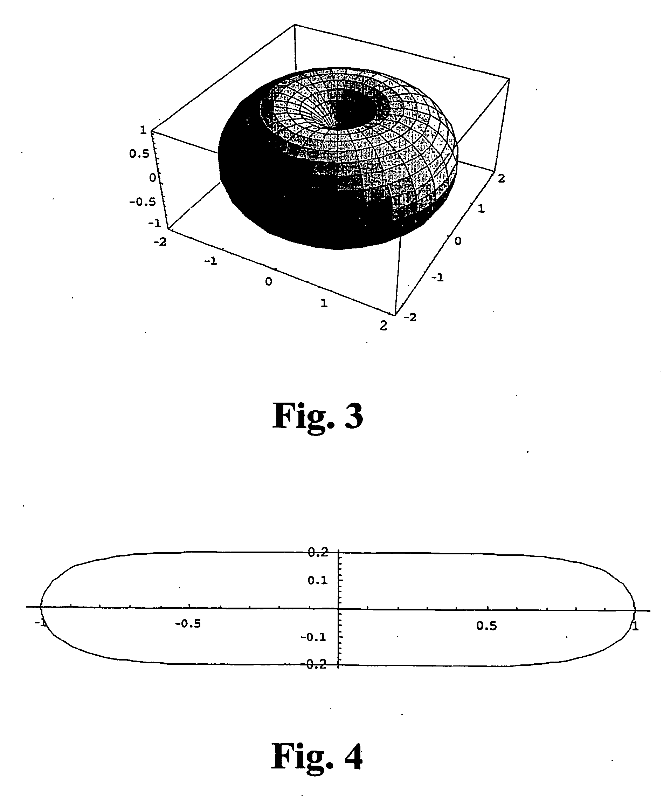

A necessary requirement for inductive coupling is that the spacing between the coils in the sensor and the transceiver is much smaller than the wavelength of the electromagnetic radiation emitted by the power coil and that the magnetic field lines of the power coil have a component that is perpendicular to the plane of the passive coil. The best coupling is obtained if the two coupling coils are of the same diameter and placed right on top of each other. Let the planes defined by the two coils be parallel. If the passive coil is moved in various directions we may obtain a sensitivity diagram similar to the radiation pattern of a dipole antenna as illustrated in Fig. 3. The specific shape of the pattern may be tailored by shaping the coils, e.g. by having an elliptic shape the acceptance angle may be larger in the horizontal plane than in the vertical plane.

If the distance between the sensor and the transceiver is larger than the wavelength the coupling is based on electromagnetic interaction like in radio antennas. Accordingly, the wireless reading of the sensor signal may be based on electromagnetic waves.

In an embodiment of the present invention, the sensor antenna is omni directional. The transceiver antenna may be selected from the group consisting of a dipole; a modified dipole; a synthesized antenna based on materials with special electromagnetic properties like a ceramic material and/or a meta material; a synthesized antenna comprising one or more resonators; and an antenna array comprising phase delays to obtain the desired directional properties.

The resonators may be split-ring resonators. The meta material may be formed as a set or an array of split-ring resonators.

If the transceiver has a simple dipole antenna with an antenna length smaller than the wavelength a toroidal radiation pattern is observed. This is also is called the Gain of the antenna. The axis of the toroid centers about the dipole. The cross section of the torus is circular. If the length of the dipole is increased a flattened pattern may be observed. This is desirable for the present application.

More sophisticated antenna designs may be incorporated as can be found in standard literature on antenna design (see for example Johnson, Richard C (Ed) "Antenna Engineering Handbook (3rd Edition)", 1993 McGraw-Hill, Chapter 4 section 2 and 8). High dielectric materials may be incorporated in order to reduce the physical size of the antennas, because a high dielectric constant will reduce the effective wavelength at the antenna. Novel materials like meta materials may be applied to obtain an even further reduction in size. Meta materials, which incorporates structures that can be in the form of locally conducting resonators allows for arbitrary designs for the permittivity and the permeability including negative values at specific frequencies.

The transceiver may be designed so as primarily to be sensitive, i.e. read the sensor signal, in a horizontal plane.

In an embodiment of the present invention, the transceiver may discard measurements if the coupling between the sensor and the transceiver is not sufficient, e.g. below a certain threshold value, indicating that the sensor is outside of the predetermined space. Thus, the transceiver may be adapted to detect presence/absence of the sensor in the predetermined space and record measurements accordingly. The measured values of the blood pressure may be adjusted in accordance with the position of the transceiver and/or the depending on the position of the patient, e.g. whether the patient is lying down or standing up.

In an embodiment of the invention, a desirable gain pattern of the transceiver is that of a torus with a cross-section of a super ellipse.

The vertical position of the sensor in relation to the heart is of importance to the accuracy of the blood pressure measurement. In an embodiment, the predetermined space covers a height range of from 15 cm below to 15 cm above the heart of the patient. Other height ranges may lead to a satisfying result. The radius of the predetermined space may range from 0 to about 1 m, preferably from about 5 cm to about 50 cm.

The wireless reading of the sensor signal may in an embodiment be based on ultrasound.

The coupling antennas may be designed in such a way that the antenna characteristics ensures that only when the sensor(s) is/are in a position within a predetermined space relative to the heart will a recording of the blood pressure actually take place.

### BRIEF DESCRIPTION OF THE DRAWINGS

The present invention will now be described in further detail with reference to the accompanying drawings, wherein:
Fig. 1 shows an embodiment of the system according to the invention,
Fig. 2 illustrates operation of the transceiver and sensors within and outside a measurement space,
Fig. 3 illustrates the gain pattern of a dipole, and
Fig. 4 illustrates a part of a cross section of a gain pattern of a dipole.

### DETAILED DESCRIPTION OF THE INVENTION

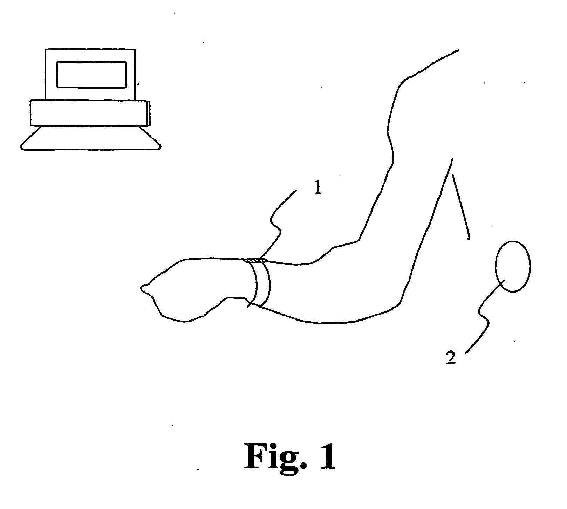

Fig. 1 shows an embodiment of the system according to the invention. The system comprises a sensor 1 having a sensor antenna, the sensor being adapted to be mounted in close proximity to an artery on the wrist of the patient. The sensor emits a sensor signal via the sensor antenna in response to the blood pressure in the artery, and the sensor antenna in the sensor allows for wireless reading of the sensor signal. Further, the system comprises a transceiver 2 that is placed on the surface of the patient in close proximity to the heart. The transceiver 2 has a transceiver antenna and is adapted to wirelessly read the sensor signal from a position close to the heart of the patient. The antenna properties of the sensor antenna and the transceiver antenna are designed so that a reading of the sensor signal can only be performed when the sensor is within a predetermined space relative to the transceiver.

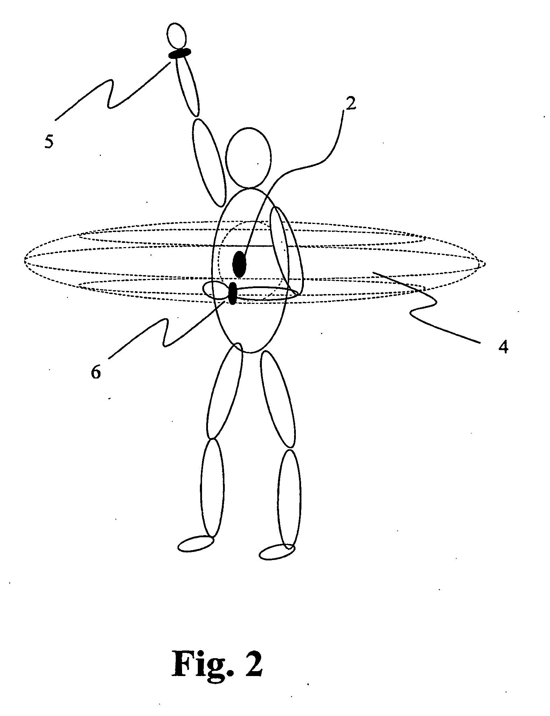

The sensor antenna has an omnidirectional gain pattern and the transceiver antenna used for reading the state of the sensor, i.e. the sensor signal, has a gain pattern implying that no signal is obtained if the sensor is not positioned within the desired, predetermined space around the transceiver, e.g. if the elevation of the sensor is very different from the elevation of the transceiver as illustrated in Fig. 2.

Fig. 2 illustrates the predetermined space 4 where the transceiver 2 reads the sensor signal from the sensor. The sensor 5 is positioned outside the space 4 and the sensor 6 is positioned inside the space, i.e. the sensor 5 is not read by the transceiver 2 and the sensor 6 may be read by the transceiver 2. Thus, the number of defective measurements from the sensor 1, 5, 6 is reduced, since the transceiver 2 does not read the sensor signal when the sensor is in a position that leads to defective measurements.

Fig. 3 shows the gain pattern of a dipole. The gain pattern of the dipole forms a torus, which can be flattened or altered by modifying the dipole. Fig. 4 shows the cross section of one ring of the torus of an antenna gain pattern. The cross section of the torus may be elliptic.

## Claims

1. A system for measuring blood pressure in an artery by wireless reading of a sensor comprising:
a sensor (1) having a sensor antenna, the sensor being adapted to be mounted in close proximity to an artery and emitting a sensor signal in response to the blood pressure in the artery, and wherein the sensor antenna allows for wireless reading of the sensor signal, and
a transceiver (2) adapted to be attached to the patient such that the position of the transceiver in relation to the heart is well defined, wherein the transceiver has a transceiver antenna and is adapted to wirelessly read the sensor signal from a position close to the heart of the patient,
wherein antenna properties of the sensor antenna and the transceiver antenna are designed so that a reading of the sensor signal can only be performed when the sensor is within a predetermined space relative to the transceiver.

2. A system according to claim 1, wherein the sensor antenna is designed to have a direction independent gain and the transceiver antenna is designed to have a directional gain which provides for a large sensitivity when the sensor is within a predetermined space relative to the transceiver and a small sensitivity when the sensor is outside this space.

3. A system according to any of the claims 1-2, wherein the wireless reading of the sensor signal is based on inductive coupling between the sensor and the transceiver.

4. A system according to claim 3, wherein a transceiver coil of the transceiver antenna is shaped so that the desired antenna characteristic is attained.

5. A system according to claim 4, wherein the transceiver coil has a loop shape with the largest dimension substantially parallel to the longitudinal axis of the patient.

6. A system according to claim 5, wherein the antenna characteristic has a cross section substantially forming an ellipse or a higher order ellipse.

7. A system according to any of the preceding claims, wherein the wireless reading of the sensor signal is based on electromagnetic waves.

8. A system according to claim 7, wherein the sensor antenna is omni directional, and the transceiver antenna is selected from the group consisting of a dipole; a modified dipole; a synthesized antenna based on materials with special electromagnetic properties like a ceramic material and/or a meta material; a synthesized antenna comprising one or more split-ring resonators; and an antenna array comprising phase delays to obtain the desired directional properties.

9. A system according to any of the claims 1-2, wherein the wireless reading of the sensor signal is based on ultrasound and the transceiver is designed so as primarily to be sensitive in a horizontal plane.

## Patentansprüche

1. System zur Messung des Blutdrucks in einer Arterie durch drahtloses Ablesen eines Sensors, umfassend:
einen Sensor (1) mit einer Sensorantenne, wobei der Sensor zum Anbringen im nahen Bereich einer Arterie und zum Aussenden eines Sensorsignals als Antwort auf den Blutdruck in der Arterie ausgebildet ist, und wobei die Sensorantenne ein drahtloses Ablesen des Sensorsignals ermöglicht, und
einen Transceiver (2), welcher zum Anbringen am Patienten derart ausgebildet ist, dass die Position des Transceivers relativ zum Herzen klar definiert ist, wobei der Transceiver eine Transceiverantenne aufweist und zum drahtlosen Ablesen des Sensorsignals aus einer in der Nähe des Herzen des Patienten befindlichen Position ausgebildet ist,
wobei die Antenneneigenschaften der Sensorantenne und der Transceiverantenne derart ausgebildet sind, dass ein Ablesen des Sensorsignals nur dann durchgeführt werden kann, wenn sich der Sensor innerhalb von einem relativ zum Transceiver vorgegebenen Bereich befindet.

2. System nach Anspruch 1, wobei die Sensorantenne mit einer richtungsunabhängigen Verstärkung und die Transceiverantenne mit einer richtungsbestimmten Verstärkung ausgebildet ist, welches, wenn sich der Sensor innerhalb von einem relativ zum Transceiver vorgegebenen Bereich befindet, eine hohe Empfindlichkeit und, wenn sich der Sensor außerhalb von diesem Bereich befindet, eine niedrige Empfindlichkeit gewährleistet.

3. System nach einem der Ansprüche 1 bis 2, wobei das drahtlose Ablesen des Sensorsignals auf einer induktiven Kopplung zwischen dem Sensor und dem Transceiver basiert.

4. System nach Anspruch 3, wobei eine Transceiverspule der Transceiverantenne in einer solchen Weise geformt ist, dass die gewünschte Antennencharakteristik erreicht ist.

5. System nach Anspruch 4, wobei die Transceiverspule eine Schleifenform besitzt, deren größte Dimension im Wesentlichen parallel zur Längsachse des Patienten ist.

6. System nach Anspruch 5, wobei die Antennencharakteristik einen im Wesentlichen eine Ellipse oder eine Ellipse höherer Ordnung bildenden Querschnitt aufweist.

7. System nach einem der vorgehenden Ansprüche, wobei das drahtlose Ablesen des Sensorsignals auf elektromagnetischen Wellen basiert.

8. System nach Anspruch 7, wobei die Sensorantenne omnidirektional ist und die Transceiverantenne ausgewählt ist aus der Gruppe bestehend aus: einem Dipol; einem modifizierten Dipol; einer synthetisierten Antenne auf der Basis von Materialien mit besonderen elektromagnetischen Eigenschaften wie zum Beispiel ein keramisches Material und/oder ein Metamaterial; einer synthetisierten Antenne umfassend einen oder mehrere Split-Ring-Resonatoren; und einem Gruppenstrahler umfassend Phasenverzögerungen zur Erzielung der gewünschten Richtungseigenschaften.

9. System nach einem der Ansprüche 1 bis 2, wobei das drahtlose Ablesen des Sensorsignals auf Ultraschall basiert und der Transceiver dazu ausgestaltet ist, vorzugsweise in einer horizontalen Ebene empfindlich zu sein.

## Revendications

1. Système de mesure de la pression sanguine dans une artère par lecture sans fil d'un capteur, comprenant :
un capteur (1) ayant une antenne pour capteur, ledit capteur étant adapté à être monté à proximité étroite d'une artère et émettant un signal capteur en réponse de la pression sanguine dans l'artère, et dans lequel l'antenne pour capteur permet lecture sans fil du signal capteur ; et
un émetteur-récepteur (2) adapté à être attaché au patient de manière à ce que la position de l'émetteur-récepteur relative au coeur est bien définie, ledit l'émetteur-récepteur ayant une antenne pour émetteur-récepteur et étant adapté à la lecture sans fil du signal capteur d'une position à proximité du coeur du patient ;
dans lequel système les propriétés de l'antenne pour capteur et l'antenne pour émetteur-récepteur sont conçues de manière à ce qu'une lecture du signal capteur soit possible uniquement quand le capteur est situé dans une espace prédéterminée relative à l'émetteur-récepteur.

2. Système selon la revendication 1, dans lequel l'antenne capteur est conçue pour avoir un gain indépendant de direction et l'antenne émetteur-récepteur est conçue pour avoir un gain directionnel qui permet une grande sensitivité quand le capteur est situé dans une espace prédéterminée relative à l'émetteur-récepteur et une grande sensitivité quand le capteur est situé en dehors de cette espace.

3. Système selon l'une quelconque des revendications 1 à 2, dans lequel la lecture sans fil du signal capteur est basée sur couplage inductif entre le capteur et l'émetteur-récepteur.

4. Système selon la revendication 3, dans lequel une bobine émetteur-récepteur de l'antenne émetteur-récepteur est façonnée de manière à ce que la caractéristique désirée de l'antenne soit obtenue.

5. Système selon la revendication 4, dans lequel la bobine émetteur-récepteur est de façon boucle dont la dimension la plus grande est essentiellement parallèle à l'axe longitudinal du patient.

6. Système selon la revendication 5, dans lequel la caractéristique de l'antenne a une coupe transversale formant une ellipse ou bien une ellipse de plus grand ordre.

7. Système selon l'une quelconque des revendications précédentes, dans lequel la lecture sans fil du signal capteur se base sur des ondes électromagnétiques.

8. Système selon la revendication 7, dans lequel l'antenne capteur est omnidirectionnel et l'antenne émetteur-récepteur est sélectionné dans le groupe consistent d'un dipôle, un dipôle modifié ; une antenne synthétisée basée sur matériaux ayant des propriétés électromagnétiques particulières comme un matériau céramique et/ou un matériau méta ; une antenne synthétisée comprenant un ou plusieurs résonateurs de bague fendue ; et un rang d'antenne comprenant temps de retard de phase afin d'obtenir les propriétés directionnelles désirées.

9. Système selon l'une quelconque des revendications 1 à 2, dans lequel la lecture sans fil du signal capteur se base sur ultrason, et l'émetteur-récepteur est conçu de manière à ce qu'il est sensitif principalement dans un plan horizontal.
